(19)

Europäisches
Patentamt
European
Patent Office
Office européen
des brevets

(11) **EP 3 406 187 A1**

(12) **EUROPEAN PATENT APPLICATION**

(43) Date of publication:
**28.11.2018 Bulletin 2018/48**

(51) Int Cl.:
**A61B 5/00** (2006.01)   **A61B 5/0215** (2006.01)
**A61B 5/053** (2006.01)

(21) Application number: **17173013.8**

(22) Date of filing: **26.05.2017**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **Koninklijke Philips N.V.
5656 AE Eindhoven (NL)**

(72) Inventors:
• **DOODEMAN, Gerardus Johannes Nicolaas
5656 AE Eindhoven (NL)**
• **DE WIJS, Willem-Jan Arend
5656 AE Eindhoven (NL)**
• **KAHLERT, Joachim
5656 AE Eindhoven (NL)**

(74) Representative: **de Haan, Poul Erik et al
Philips International B.V.
Philips Intellectual Property & Standards
High Tech Campus 5
5656 AE Eindhoven (NL)**

(54) **MEASURING A PROPERTY IN A BODY**

(57) A measurement circuit for measuring a property in a body comprises: a resonant circuit comprising a first element and a second element, wherein at least one of the first element and the second element is configured such that a property thereof is dependent on the property in the body; and a non-linear element arranged to generate intermodulation products when the circuit is driven by an input signal comprising a first component at a first frequency and a second component at a second frequency. A measurement system for measuring a property in a body comprises: a measurement circuit as described above; a driving circuit for providing a driving signal to the measurement circuit, wherein the driving signal comprises a first component at a first frequency and a second component at a second frequency; and an analyzer circuit for analyzing a response of the measurement circuit in order to obtain a measurement of the property.

Figure 4

**Description**

TECHNICAL FIELD OF THE INVENTION

The invention relates to a measurement circuit, measurement system, and measurement method for measuring a property in a body.

BACKGROUND OF THE INVENTION

Pressure measurements inside the body of a human or other animal patient can be used to assess a multitude of physical ailments, such as coronary artery stenosis, liver portal vein pressure, and pulmonary vein pressure, to mention just a few. In order to be able to measure pressure, in many cases at least two wires must be integrated into a device that can be positioned inside the body, which puts conditions on the interconnection of the device outside the body.

In patent applications WO2012/109039, WO2014/164734 and WO2015/099845 a pressure sensitive resonant circuit is described. The resonant frequency of the circuit depends on pressure because it includes a pressure dependent inductance and/or capacitance. The resonant circuit is connected at one side to the human body, and on the other side to a guide wire. The resonant frequency is determined by measuring the impedance of the resonant circuit via the guidewire and the return loop of the body.

One disadvantage of this measurement method is that the signal path through the body and the guide wire are part of the resonant circuit. This means that the behavior of the resonant circuit changes with all changes in orientation of the body and guide wire and changes with drift and environmental alterations

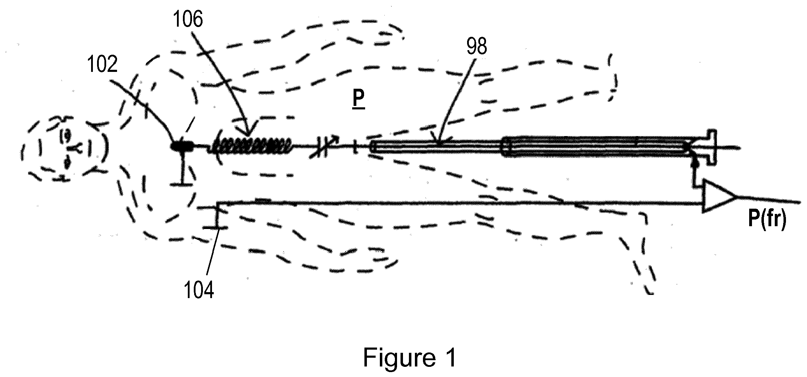

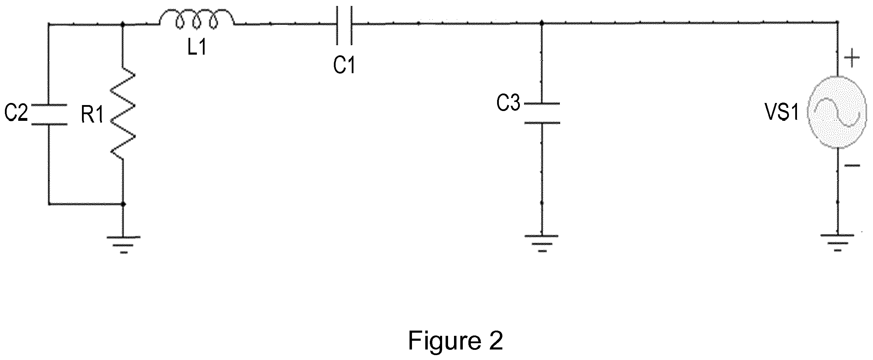

Figure 1 shows a measurement system according to patent application WO2014/164734. An inductance is connected in series with a pressure dependent capacitor to form a resonance circuit 106, and are connected to an electrode 102 and a guide wire 98 to form a circuit which is electrically equivalent to the electrical circuit shown in Figure 2. In Figure 2, C1 is the pressure dependent capacitor, L1 is the inductance in the resonant circuit 106, R1 and C2 represent the body resistance and capacitance respectively, and C3 represents the parasitic capacitance of the guide wire 98.

The resonance circuit 106 is inserted into the body of the patient P, who is connected to ground potential through a ground electrode 104.

The voltage source VS1 generates a swept frequency signal. The current delivered by the voltage source is measured and the voltage to current ratio is a measure of the impedance of the circuit as function of frequency. The resonance frequency changes as a function of pressure and the resonance frequency will also change when any parasitic element in the body return path is altered e.g. by body movement, temperature changes or drift of component properties

SUMMARY OF THE INVENTION

It is an object of the present invention to provide an improved measurement circuit, measurement system and measurement method for measuring a property in a body.

The invention provides a measurement circuit, measurement system and measurement method for measuring a property in a body to mitigate the problems in the prior art.

In a first aspect of the present invention, there is provided a measurement circuit for measuring a property in a body, the measurement circuit comprising:

a resonant circuit comprising a first element and a second element, wherein at least one of the first element and the second element is configured such that a property thereof is dependent on the property in the body; and

a non-linear element arranged to generate intermodulation products when the circuit is driven by an input signal comprising a first component at a first frequency and a second component at a second frequency.

In some embodiments, at least one of the first element and the second element is configured such that an electrical property thereof is dependent on the property in the body.

In some embodiments, the first element is an inductance and the second element is a capacitor.

The first element and the second element may be arranged in parallel.

The non-linear element may comprise two antiparallel diodes.

In some embodiments, the first element is an inductance and the second element is a capacitor, the inductance and the capacitor are arranged in parallel, and the two antiparallel diodes are connected in parallel with the inductance and the capacitor.

In some embodiments, an electrical property of at least one of the first element and the second element is dependent on pressure in the body.

In a second aspect of the present invention, there is provided a measurement system for measuring a property in a body, the measurement system comprising:

a measurement circuit according to the first aspect;

a driving circuit for providing a driving signal to the measurement circuit, wherein the driving signal comprises a first component at a first frequency and a second component at a second frequency; and

an analyzer circuit for analyzing a response of the measurement circuit in order to obtain a measurement of the property.

The driving circuit may be arranged to provide the driving signal comprising the first component at the first frequency and the second component at the second frequency, and wherein the first frequency is above a resonant frequency of the resonant circuit and the second frequency is below the resonant frequency of the resonant circuit.

The driving circuit may be arranged to provide the driving signal comprising the first component at the first frequency and the second component at the second frequency, wherein the first and the second frequency fall within the bandwidth of the resonant circuit.

In some embodiments, the analyzer circuit comprises a frequency analyzer. The analyzer circuit may be configured to measure a ratio of the amplitudes of odd order intermodulation products resulting from the first and second components of the driving signal.

The measurement system may further comprise a wire connection connecting the driving circuit and the measurement circuit.

In other embodiments, the measurement system may further comprise a transmitter arranged to wirelessly transmit the driving signal from the driving circuit to the measurement circuit.

In some embodiments, the system is arranged to wirelessly transmit the response of the measurement circuit to the analyzer circuit.

In a third aspect of the present invention, there is provided a method of measuring a property in a body, the method comprising:

applying a driving signal to a measurement circuit, wherein the driving signal comprises a first component at a first frequency and a second component at a second frequency, and wherein the measurement circuit comprises:

a resonant circuit comprising a first element and a second element, wherein at least one of the first element and the second element is configured such that a property thereof is dependent on the property in the body; and a non-linear element arranged to generate intermodulation products when the circuit is driven by an input signal comprising a first component at a first frequency and a second component at a second frequency; and analyzing a response of the measurement circuit in order to obtain a measurement of the property.

These and other aspects of the invention will be apparent from and elucidated with reference to the embodiments described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 shows a measurement system according to the prior art;
Figure 2 shows an equivalent schematic circuit diagram of the measurement system of Figure 1;
Figure 3 illustrates an embodiment of the measurement system according to the invention;
Figure 4 illustrates a further embodiment of the measurement system according to the invention;
Figure 5 shows signals generated in an embodiment of the measurement system according to the invention;
Figure 6 shows further signals generated in an embodiment of the measurement system according to the invention;
Figure 7 illustrates an effect of a shift in resonant frequency in an embodiment of the measurement system according to the invention; and
Figure 8 is a flow chart illustrating a method according to an aspect of the invention.

DETAILED DESCRIPTION OF PREFERRED EMBODIMENTS

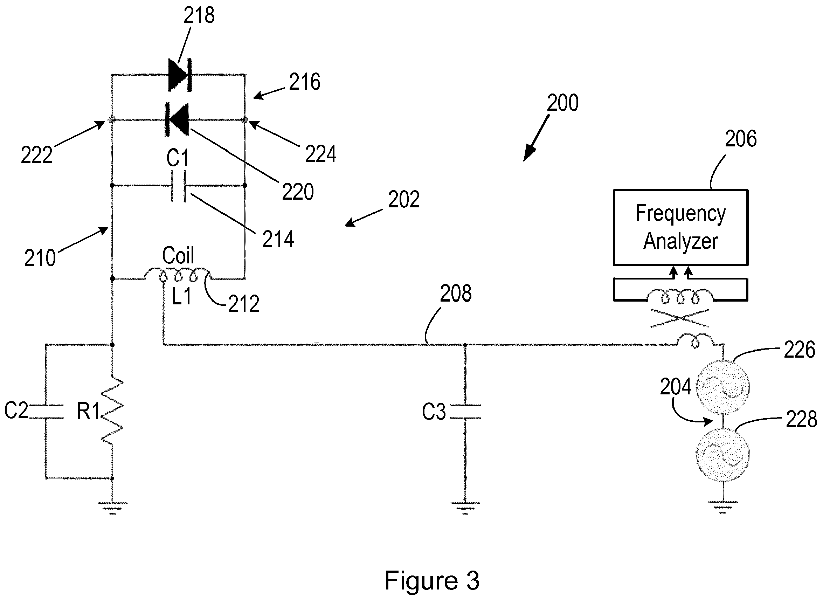

Figure 3 illustrates a first embodiment of a measurement system in accordance with the invention.

The measurement system 200 shown in Figure 3 comprises a measurement circuit 202, a driving circuit 204 for providing a driving signal to the measurement circuit 202, and a frequency analyzer circuit 206.

The measurement circuit 202 is connected to a guide wire 208, which connects the measurement circuit 202 to the driving circuit 204 and the frequency analyzer circuit 206, and is also used to guide the measurement circuit 202 to the intended operating location in the body of a human or animal patient.

The measurement circuit 202 includes a resonant circuit 210, which comprises a coil 212 having an inductance L1 and a capacitor 214 having a capacitance C1. In this illustrated embodiment, the inductor 212 and the capacitor 214 are connected in parallel. In other embodiments, an inductor and a capacitor are connected in series. At least one of the inductor 212 and the capacitor 214 is configured such that an electrical property thereof is dependent on a property within the body that is intended to be measured.

In one embodiment, the capacitor 214 is configured such that its capacitance is dependent on a pressure at the operating location within the body. For example, the capacitor 214 may contain a flexible, impressionable dielectric layer.

In other embodiments, the resonant circuit 210 includes an inductor whose inductance is dependent on the pressure. For example, the inductor may be bendable, or may include an impressionable coil, or a coil with a pressure-sensitive movable conductive or magnetic core.

In other embodiments, at least one of the inductor and the capacitor is configured such that an electrical property thereof is dependent on a different property within the body, for example such as the permittivity or permeability of bodily fluids such as blood, allowing variations in one of these properties to be measured.

The measurement circuit 202 further includes a non-linear element 216. In this illustrated embodiment, the non-linear element 216 comprises first and second diodes 218, 222 connected in anti-parallel. That is, current can flow from the node 222 to the node 224 in the forward direction of the first diode 218, and can flow from the node 224 to the node 222 in the forward direction of the second diode 220.

The electrical circuits can be made according to methods described in International Patent Applications WO2017/013224 and WO2016/207041, where transducer circuits are constructed using pressure electrical contacts and isolating adhesive layers. These methods, and in particular, the methods of integrating and interconnecting electrical elements (transducers) to wiring in a form factor suitable for guide wires, can also be used to construct pressure dependent capacitors.

Alternatively, the transducer may be replaced by an air capacitor and thin diodes (<150 $\mu$m thick, preferably <50$\mu$m thick, and most preferably <15$\mu$m thick), and the wires used for interconnection can form the coil structure and potentially an antenna structure, all in a form factor of <200$\mu$m, preferably <100$\mu$m, and most preferably <50$\mu$m thick, with the consequence that integration in a guide wire or implantable device is feasible.

Figure 3 also shows the electrical effect of the measurement circuit 202, in use. Specifically, Figure 3 shows that, when the measurement circuit 202 is at its intended operating location in the body of a human or animal patient, the body has an effective resistance R1 and an effective capacitance C2. The guide wire 208 also has a parasitic capacitance C3. The effective resistance R1 and the effective capacitance C2 of the body are sensitive to movement of the body, amongst other things.

Therefore, in the embodiment shown in Figure 3, the resonant circuit 210 and the non-linear element 216 are isolated from the body resistance R1 and capacitance C2, in order to reduce the effect of any changes in the body resistance R1 and capacitance C2 on the resonant frequency of the resonant circuit 210.

Specifically, the guide wire 208 is connected to the resonant circuit 210 at a tap of the coil 212. This provides a good degree of isolation of the variable guide wire impedance from the resonant circuit.

The driving circuit 204 is configured to generate a driving signal comprising a first component at a first frequency and a second component at a second frequency. This is represented in Figure 3 by a first driving signal source 226 and a second driving signal source 228.

The operation of the driving circuit 204 is described in more detail below.

The frequency analyzer circuit 206 detects the response of the measurement circuit 202 to the driving signal.

The operation of the frequency analyzer circuit 206 is described in more detail below.

A resistance can be included in the guide wire 208 or in the measurement circuit 210 where it connects to the guide wire, in order to prevent excessive current being driven into the body.

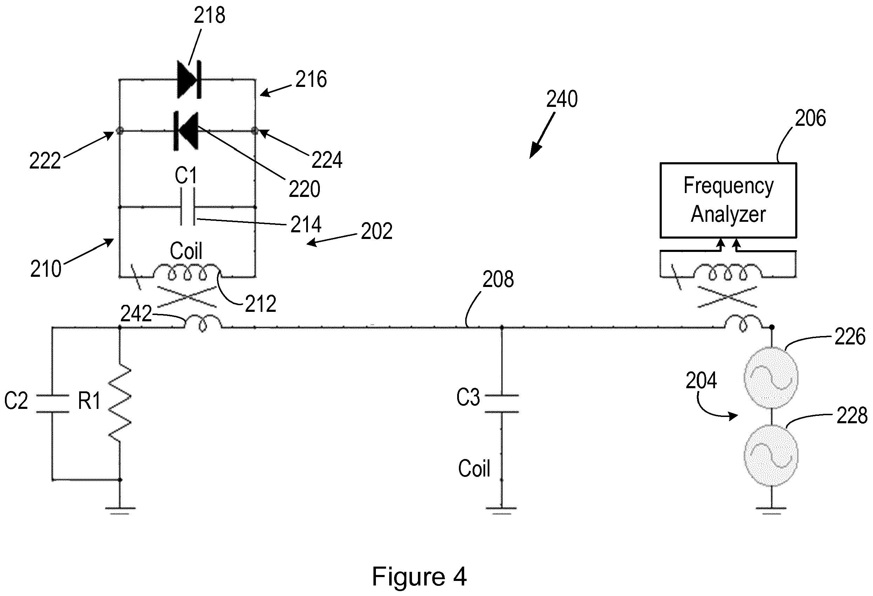

Figure 4 illustrates a second embodiment of a measurement system in accordance with the invention.

The measurement system 240 shown in Figure 4 is similar in some respects to the measurement system 200 shown in Figure 3, and the same reference numerals are used to indicate features that are the same. Thus, the measurement system 240 shown in Figure 4 comprises a measurement circuit 202, a driving circuit 204 for providing a driving signal to the measurement circuit 202, and a frequency analyzer circuit 206.

The measurement circuit 202 is connected to a guide wire 208, which connects the measurement circuit 202 to the driving circuit 204 and the frequency analyzer circuit 206, and is also used to guide the measurement circuit 202 to the intended operating location in the body of a human or animal patient.

The measurement circuit 202 includes a resonant circuit 210, which comprises a coil 212 having an inductance L1 and a capacitor 214 having a capacitance C1. In this illustrated embodiment, the inductor 212 and the capacitor 214 are connected in parallel. In other embodiments, an inductor and a capacitor are connected in series. At least one of the inductor 212 and the capacitor 214 is configured such that an electrical property thereof is dependent on a property within the body that is intended to be measured.

In one embodiment, the capacitor 214 is configured such that its capacitance is dependent on a pressure at the operating location within the body. For example, the capacitor 214 may contain a flexible, impressionable dielectric layer.

In other embodiments, the resonant circuit 210 includes an inductor whose inductance is dependent on the pressure. For example, the inductor may be bendable, or may include an impressionable coil, or a coil with a pressure-sensitive movable conductive or magnetic core.

In other embodiments, at least one of the inductor and the capacitor is configured such that an electrical property thereof is dependent on a different property within the body, for example such as the permittivity or permeability of bodily fluids such as blood, allowing variations in one of these properties to be measured.

The measurement circuit 202 further includes a non-linear element 216. In this illustrated embodiment, the non-linear element 216 comprises first and second diodes 218, 222 connected in anti-parallel. That is, current can flow from the node 222 to the node 224 in the forward direction of the first diode 218, and can flow from the node 224 to the node 222 in the forward direction of the second diode 220.

The electrical circuits can be made according to methods described in International Patent Applications WO2017/013224 and WO2016/207041, where transducer circuits are constructed using pressure electrical contacts and isolating adhesive layers. These methods, and in particular, the methods of integrating and interconnecting electrical elements (transducers) to wiring in a form factor suitable for guide wires, can also be used to construct pressure dependent capacitors.

Alternatively, the transducer maybe replaced by an air capacitor and thin diodes (<150 $\mu$m thick, preferably <50$\mu$m thick, and most preferably <15$\mu$m thick), and the wires used for interconnection can form the coil structure and potentially an antenna structure, all in a form factor of <200$\mu$m, preferably <100$\mu$m, and most preferably <50$\mu$m thick, with the consequence that integration in a guide wire or implantable device is feasible.

Figure 4 also shows the electrical effect of the measurement circuit 202, in use. Specifically, Figure 4 shows that, when the measurement circuit 202 is at its intended operating location in the body of a human or animal patient, the body has an effective resistance R1 and an effective capacitance C2. The guide wire 208 also has a parasitic capacitance C3. The effective resistance R1 and the effective capacitance C2 of the body are sensitive to movement of the body, amongst other things.

Therefore, in the embodiment shown in Figure 4, the resonant circuit 210 and the non-linear element 216 are isolated from the body resistance R1 and capacitance C2, in order to reduce the effect of any changes in the body resistance R1 and capacitance C2 on the resonant frequency of the resonant circuit 210.

Specifically, the guide wire 208 includes an inductor 242, which has an inductive connection to the coil 212. Because of the very low currents flowing through the guide wire, due to the high resistance R1 and low capacitance C2, the mutual inductance will cause minimal effects on the resonant frequency of the resonant circuit.

The driving circuit 204 is configured to generate a driving signal comprising a first component at a first frequency and a second component at a second frequency. This is represented in Figure 3 by a first driving signal source 226 and a second driving signal source 228.

The operation of the driving circuit 204 is described in more detail below.

The frequency analyzer circuit 206 detects the response of the measurement circuit 202 to the driving signal.

The operation of the frequency analyzer circuit 206 is described in more detail below.

A resistance can be included in the guide wire 208 or in the measurement circuit 210 where it connects to the guide wire, in order to prevent excessive current being driven into the body.

Thus, Figures 3 and 4 shows embodiments in which the driving signal is applied in a wired manner to the resonance circuit, and the response of the resonance circuit is returned in a wired manner to the frequency analyzer.

In other embodiments, the driving signal is applied wirelessly to the resonance circuit and/or the response is returned wirelessly to the frequency analyzer. For example, the driving signal can be transmitted to the resonance circuit via an electrical conductor in a guidewire, with the return path for the response being provided by the patient body. The contact between the guide wire and the measurement circuit may be made by means of a brush contact (as the exact value of the contact resistance is not critical). For a body return path, a standard electrocardiogram (ECG) lead can be connected to the system (or patched through).

In some embodiments, the driving signal comprising the two frequencies can also be sent wirelessly through the body to the guide wire, and the intermodulation products sent back also via the guide wire, using it as an antenna, with suitable choice of driving and transmission frequencies.

In other embodiments, the two frequency components of the driving signal can be transmitted using ultrasonic transducers (as described in US 2013/0060139 A1) and converted to electrical signals using for example a polyvinylidene fluoride (pvdf) transducer or a piezoelectric transducer (PZT) to avoid having to also receive reflected ultrasonic energy, but to receive the transduced electrical energy through the guide wire acting as antenna.

Similarly, in other embodiments, a similar system can be constructed using optical frequencies and photo-diodes.

The embodiments shown in Figures 3 and 4, and the further embodiments described above, operate in generally the same way. As mentioned above, the driving circuit 204 is configured to generate a driving signal comprising a first component at a first frequency and a second component at a second frequency. The first and second components are preferably sinusoidal signals. The use of the two sinusoidal signals at discrete frequencies avoids the need to sweep through a frequency range of interest whenever a measurement is required.

As described in more detail below, in some embodiments, the first and second frequencies are set up so that the baseline resonant frequency of the resonant circuit 210 is midway between them. Thus, to set this up, the resonant circuit is first excited by a frequency scan across the expected range of the resonant frequency. This range should be relatively narrow, and so the frequency scan will not need to cover a wide range of frequencies. This allows the baseline resonant frequency to be determined. Then the first frequency and the second frequency to be generated by the driving circuit in use are adjusted so that the baseline resonance frequency is midway between them.

The baseline resonant frequency, and the first frequency and the second frequency, may be at radio frequencies, for example between 50MHz and 2GHz.

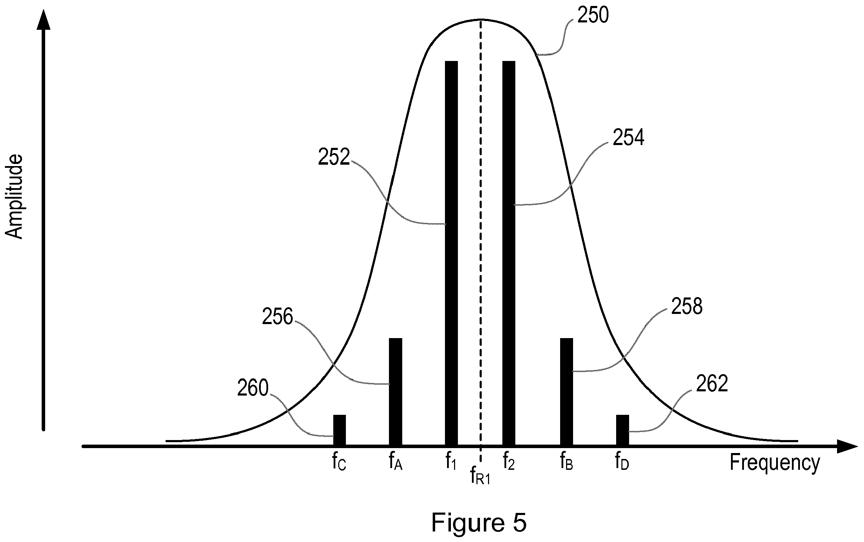

Figure 5 shows the effect of this operation, in a situation where the resonance curve of the resonant circuit 210 is illustrated by the line 250. The resonant circuit 210 has a baseline resonant frequency, for example at a time before the measurement circuit is inserted into the body of the patient, of $f_{R1}$. When the resonant circuit 210 is excited by a signal at this resonant frequency $f_{R1}$, it produces a response having the maximum power. When the resonant circuit 210 is excited by a signal at a different frequency, it produces a response having a lower power, which is determined by the shape of the resonance curve 250.

The resonant frequency $f_{R1}$ of the measurement circuit 202 depends on the inductance L1 of the inductor 212 and the capacitance C1 of the capacitor 214. Specifically:

$$f_{R1} = \frac{1}{2\pi\sqrt{L1 \bullet C1}}\,.$$

For an example, if the inductance L1 of the inductor 212 is equal to 20nH, and the capacitance C1 of the capacitor 214 is equal to 5pF, then the resonant frequency $f_{R1}$ of the measurement circuit 202 is approximately 500MHz.

Specifically, Figure 5 shows the situation when the driving circuit 204 is configured to generate a driving signal comprising a first component at a first frequency $f_1$ and a second component at a second frequency $f_2$. The first frequency $f_1$ and the second frequency $f_2$ are chosen such that the baseline resonant frequency $f_{R1}$ is between the first frequency $f_1$ and the second frequency $f_2$.

The first frequency $f_1$ and the second frequency $f_2$ are chosen in this example such that they differ by less than 10%. More specifically, the first frequency $f_1$ and the second frequency $f_2$ are chosen in this example such that they differ by less than 1%. More specifically still, the first frequency $f_1$ and the second frequency $f_2$ are chosen in this example such that they differ by less than 0.1%.

As an illustrative example, if the resonant frequency $f_{R1}$ of the measurement circuit 202 is approximately 500 MHz, the first frequency $f_1$ and the second frequency $f_2$ may be chosen such that they differ by 100 kHz, which is 0.02%.

The quality factor (Q factor) of the resonant circuit 210 depends on the effective resistance of the resonant circuit. This effective resistance is reduced in the embodiments shown in Figures 3 and 4, in which the resonant circuit 210 is isolated from the parasitic resistance of the body. Reducing the effective resistance has the effect of increasing the Q factor of the resonant circuit 210 and thus reducing the effective bandwidth of the circuit, which may be defined as the frequency difference between the two frequencies at which the power of the response of the resonant circuit is one half of the peak power of the response at the resonant frequency.

The first frequency $f_1$ and the second frequency $f_2$ are chosen in this example such that they are both within the effective bandwidth of the resonant circuit 210.

Thus, Figure 5 shows that the measurement circuit 202 produces a response 252 to the first component of the driving signal at the first frequency $f_1$ and produces a response 254 to the second component of the driving signal at the second frequency $f_2$. Where the first frequency $f_1$ and the second frequency $f_2$ are chosen such that the baseline resonant frequency $f_{R1}$ is midway between the first frequency $f_1$ and the second frequency $f_2$, as shown by way of illustration in Figure 5, the response 252 and the response 254 are of similar magnitude.

The effect of including the non-linear element 216 in the measurement circuit 202 is that its response also includes harmonics and intermodulation components. By choosing the first and second frequencies to be close to the resonant frequency of the measurement circuit 202, the harmonics, at multiples of the first and second frequencies, and the even-order intermodulation components are effectively filtered out by the frequency-dependent measurement circuit 202, and so, in the bandwidth of interest, it is the odd-order intermodulation components that are relevant. The resonant circuit 210 also acts as a narrowband bandpass system that has the effect of suppressing out of band noise.

Figure 5 illustrates the third-order intermodulation components. Specifically, Figure 5 illustrates a first third-order intermodulation component 256 at the frequency $f_A$ (= $2.f_1 - f_2$) and a second third-order intermodulation component 258 at the frequency $f_B$ (= $2.f_2 - f_1$).

Since the baseline resonant frequency $f_{R1}$ is midway between the first frequency $f_1$ and the second frequency $f_2$, as shown by way of illustration in Figure 5, the first third-order intermodulation component 256 and the second third-order intermodulation component 258 are of similar magnitude.

Since the first frequency $f_1$ and the second frequency $f_2$ are close together, the frequency $f_A$ of the first third-order intermodulation component 256 and the frequency $f_B$ of the second third-order intermodulation component 258 are both within (or close to) the effective bandwidth of the resonant circuit 210.

Figure 5 also illustrates the fifth-order intermodulation components. Specifically, Figure 5 illustrates a first fifth-order intermodulation component 260 at the frequency fc (= $3.f_1 - 2.f_2$) and a second fifth-order intermodulation component 262 at the frequency $f_D$ (= $3.f_2 - 2.f_1$).

Since the baseline resonant frequency $f_{R1}$ is midway between the first frequency $f_1$ and the second frequency $f_2$, as shown by way of illustration in Figure 5, the first fifth-order intermodulation component 260 and the second fifth-order intermodulation component 262 are of similar magnitude.

If the first frequency $f_1$ and the second frequency $f_2$ are sufficiently close together, the frequency fc of the first fifth-order intermodulation component 260 and the frequency $f_D$ of the second fifth-order intermodulation component 262 are both within (or close to) the effective bandwidth of the resonant circuit 210.

When the measurement circuit 202 is inserted into the body of the patient, an electrical property of the resonant circuit may change. As described previously, the resonant circuit comprises a first element and a second element, and at least one of the first element and the second element is configured such that an electrical property thereof is dependent on a property in the body. In some embodiments, the resonant circuit comprises a capacitor and an inductor, and the capacitor is configured such that its capacitance is dependent on a pressure in the body.

If the capacitance changes due to the pressure in the body, the resonant frequency of the resonant circuit changes.

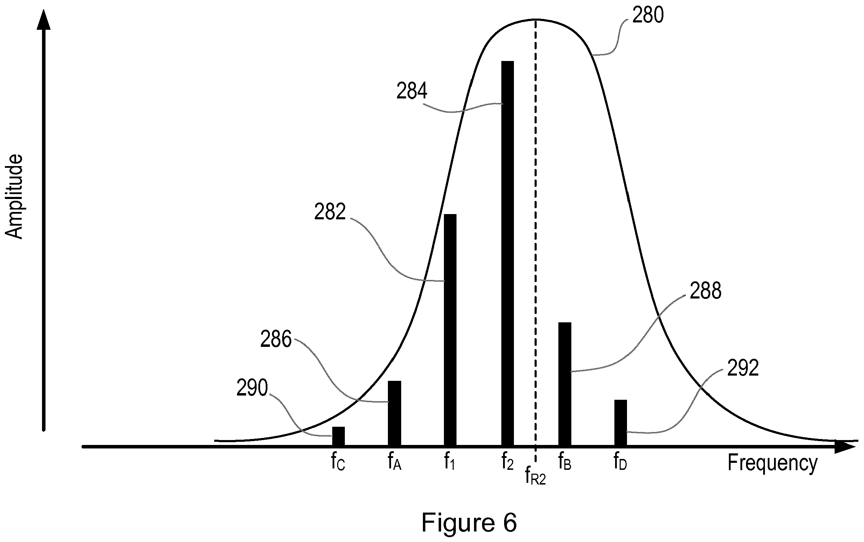

Figure 6 shows the effect of this operation, in a situation where the resonance curve of the resonant circuit 210 is illustrated by the line 280.

The resonant frequency $f_{R2}$ still depends on the inductance of the inductor 212 and the capacitance of the capacitor 214, but the change in the capacitance due to the pressure in the body means that the resonant frequency of the resonant circuit has changed.

Figure 6 shows the situation when, as illustrated in Figure 5, the driving circuit 204 is configured to generate a driving signal comprising a first component at a first frequency $f_1$ and a second component at a second frequency $f_2$. The new resonant frequency $f_{R2}$ is higher than the second frequency $f_2$.

Thus, Figure 6 shows that the measurement circuit 202 produces a response 282 to the first component of the driving signal at the first frequency $f_1$ and produces a response 284 to the second component of the driving signal at the second frequency $f_2$. Because the new resonant frequency $f_{R2}$ is higher than the second frequency $f_2$, as shown by way of illustration in Figure 5, the response 284 is larger than the response 282.

As before, the effect of including the non-linear element 216 in the measurement circuit 202 is that its response also includes intermodulation components, and it is the odd-order intermodulation components that are relevant.

Figure 6 illustrates the third-order intermodulation components. Specifically, Figure 6 illustrates a first third-order intermodulation component 286 at the frequency $f_A$ (= $2.f_1$- $f_2$) and a second third-order intermodulation component 288 at the frequency $f_B$ (= $2.f_2$ - $f_1$).

Since the new resonant frequency $f_{R2}$ is higher than the second frequency $f_2$, as shown by way of illustration in Figure 6, the second third-order intermodulation component 288 has a larger magnitude than the first third-order intermodulation component 286.

If the first frequency $f_1$ and the second frequency $f_2$ are sufficiently close together, the frequency $f_A$ of the first third-order intermodulation component 286 and the frequency $f_B$ of the second third-order intermodulation component 288 are both within (or close to) the effective bandwidth of the resonant circuit 210.

Figure 6 also illustrates the fifth-order intermodulation components. Specifically, Figure 6 illustrates a first fifth-order intermodulation component 290 at the frequency fc (= $3.f_1$- $2.f_2$) and a second fifth-order intermodulation component 292 at the frequency $f_D$ (= $3.f_2$ - $2.f_1$).

Since the new resonant frequency $f_{R2}$ is higher than the second frequency $f_2$, as shown by way of illustration in Figure 6, the second fifth-order intermodulation component 292 has a larger magnitude than the first fifth-order intermodulation component 290.

If the first frequency $f_1$ and the second frequency $f_2$ are sufficiently close together, the frequency fc of the first fifth-order intermodulation component 290 and the frequency $f_D$ of the second fifth-order intermodulation component 292 are both within (or close to) the effective bandwidth of the resonant circuit 210.

The response of the measurement circuit 202 to the driving signals generated by the driving circuit 204 is measured by the frequency analyzer circuit 206.

Specifically, the frequency analyzer circuit 206 comprises a spectrum analyzer, which measures the amplitudes of signal components at different frequencies.

The frequency analyzer circuit 206 can therefore distinguish the intermodulation products from the components of the drive signal itself.

Based on the measured amplitudes of the signal components at the different frequencies, the frequency analyzer circuit 206 is able to determine the shift in the resonant frequency of the measurement circuit 202, and hence is able to determine the change in the value of the electrical property of the variable component of the resonant circuit. That is, when the resonant circuit contains a capacitor, whose capacitance depends on the pressure or other parameter in the body, or an inductor whose inductance depends on the pressure or other parameter in the body, the shift in the resonant frequency of the measurement circuit can be used to determine the value of the variable capacitance or inductance (as the case may be) and hence can be used to determine the value of the pressure or other parameter in the body.

For example, the shift in the resonant frequency can be determined by examining the ratio of the amplitudes of the intermodulation products.

More specifically, if the shape of the resonance curve 280 shown in Figure 6 is known, then the ratio of the amplitude of the second third-order intermodulation component 288 to the amplitude of the first third-order intermodulation component 286 will indicate the shift in the resonant frequency.

Similarly, the ratio of the amplitude of the second fifth-order intermodulation component 292 to the amplitude of the first fifth-order intermodulation component 290 will indicate the shift in the resonant frequency.

Thus, either the third-order intermodulation components, or the fifth-order intermodulation components, or similarly any higher odd-order intermodulation components that are large enough to be detected, can be used. Amplitude ratios of more than one of the odd-order intermodulation components can be found, and combined (for example, averaged) to obtain a measurement result.

This shift in the resonant frequency in turn will indicate the shift in the variable property of the variable component of the resonant circuit. Finally, knowledge of the characteristics of this variable component will allow a determination of the property to be measured in the body of the patient.

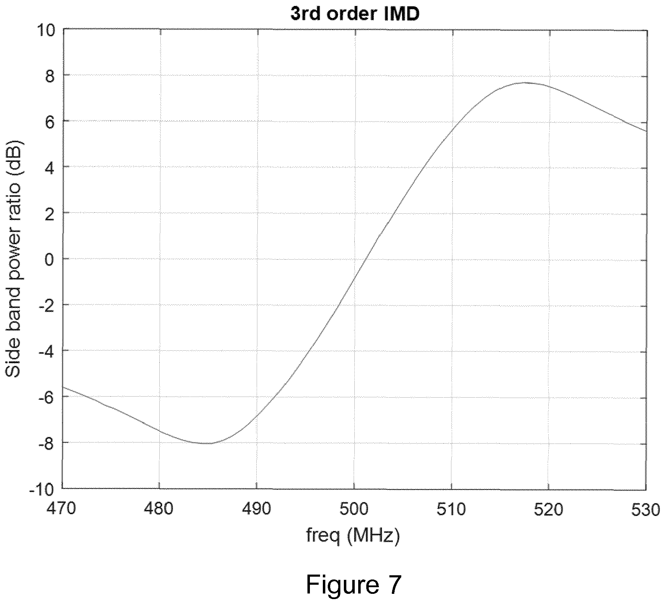

Figure 7 illustrates an example where the baseline resonant frequency is about 500 MHz. Specifically, Figure 7 illustrates the relationship between the ratio of the amplitudes of the two third-order intermodulation products and the resonant frequency. Thus, when the resonant frequency is midway between the frequencies of the driving signals generated by the driving circuit 204 (and assuming that the two driving signals generated by the driving circuit 204 have equal amplitudes), the response of the measurement circuit contains components at these two frequencies that are of equal amplitude, and hence the power ratio is 0dB. In the example illustrated in Figure 7, this occurs when the resonant frequency is about 501 MHz.

As the resonant frequency increases, the power ratio (that is the ratio of the power of the second third-order intermodulation component 288 to the power of the first third-order intermodulation component 286, as shown in Figure 6) increases. For example, when the resonant frequency is 510 MHz, the power ratio is about 6dB. Similarly, as the resonant frequency decreases, the power ratio (that is the ratio of the power of the second third-order intermodulation component 288 to the power of the first third-order intermodulation component 286, as shown in Figure 6) decreases. For example, when the resonant frequency is 490 MHz, the power ratio is about -7dB.

Thus, by measuring the power ratio, it is possible to determine the resonant frequency (provided that this varies within the range from about 485-515MHz, in this example), and hence it is possible to determine the value of the electrical property of the component of the resonant circuit that depends on the pressure or other parameter in the body. Based on knowledge of the characteristic of this component, it is then possible to determine the value of the pressure or other parameter in the body.

Similarly, the shift in the resonant frequency can be determined by examining the ratio of the amplitudes of the fifth-order intermodulation products, or any suitable higher-order intermodulation products.

The use of a ratio measurement as described here is robust against disturbances such as movements of the patient, effects due to changes in temperature, and the aging of components of the device.

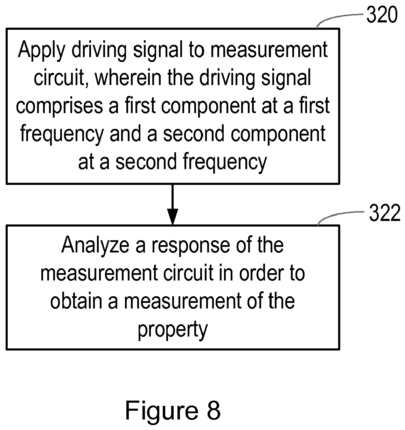

Figure 8 is a flow chart, illustrating a method of measuring a property in a body.

In step 320, a driving signal is applied to a measurement circuit. The driving signal comprises a first component at a first frequency and a second component at a second frequency. As described above, the measurement circuit comprises a resonant circuit comprising a first element and a second element, and at least one of the first element and the second element is configured such that a property thereof is dependent on the property in the body. The measurement circuit also comprises a non-linear element arranged to generate intermodulation products when the circuit is driven by an input signal comprising a first component at a first frequency and a second component at a second frequency.

In step 322, a response of the measurement circuit is analyzed in order to obtain a measurement of the property in the body.

The examples of methods described above use two different excitation frequencies to measure a variable electrical property of a measurement circuit. However, the invention is not limited to electrical fields, but can also be extended to ultrasonic or optical fields (using a non-linear element). For example, the measurement circuit can include an ultrasonic resonator with a mechanical non-linearity, which is sensitive to a property to be measured in the body, and the driving circuit can then apply ultrasonic signals, which will result in ultrasonic sidebands. As described above, the magnitudes of the intermodulation components can be used to detect the change in the resonant frequency of the resonant circuit. Also as described above, this can then be used to determine the change in the property of the resonator, and hence the change in the property to be measured in the body.

As another example, the measurement circuit can include an optical resonator with an optical non-linear element, which can generate intermodulation products when, for example, two laser beams at different wavelengths are applied to it. As described above, the magnitudes of the intermodulation components can be used to detect the change in the resonant frequency of the resonant circuit. Also as described above, this can then be used to determine the change in the property of the resonator, and hence the change in the property to be measured in the body can cause new light wave.

There is therefore provided a method and a circuit for measuring a property in a body.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1. A measurement circuit for measuring a property in a body, the measurement circuit comprising:

   a resonant circuit comprising a first element and a second element, wherein at least one of the first element and the second element is configured such that a property thereof is dependent on the property in the body; and
   a non-linear element arranged to generate intermodulation products when the circuit is driven by an input signal comprising a first component at a first frequency and a second component at a second frequency.

2. A measurement circuit according to claim 1, wherein at least one of the first element and the second element is configured such that an electrical property thereof is dependent on the property in the body.

3. A measurement circuit according to claim 2, wherein the first element is an inductance and the second element is a capacitor.

4. A measurement circuit according to claim 2 or 3, wherein the first element and the second element are arranged in parallel.

5. A measurement circuit according to any of the claims 2 to 4, wherein the non-linear element comprises two antiparallel diodes.

6. A measurement circuit according to claim 5, wherein the first element is an inductance and the second element is a capacitor, wherein the inductance and the capacitor are arranged in parallel, and wherein the two antiparallel diodes are connected in parallel with the inductance and the capacitor.

7. A measurement circuit according to any of the claims 2 to 6, wherein an electrical property of at least one of the first element and the second element is dependent on pressure in the body.

8. A measurement system for measuring a property in a body, the measurement system comprising:

   a measurement circuit according to any of the claims 1 to 7;
   a driving circuit for providing a driving signal to the measurement circuit, wherein the driving signal comprises a first component at a first frequency and a second component at a second frequency; and
   an analyzer circuit for analyzing a response of the measurement circuit in order to obtain a measurement of the property.

9. A measurement system according to claim 8, wherein the driving circuit is arranged to provide the driving signal comprising the first component at the first frequency and the second component at the second frequency, and wherein the first frequency is above a baseline resonant frequency of the resonant circuit and the second frequency is below the baseline resonant frequency of the resonant circuit.

10. A measurement system according to claim 8 or 9, wherein the driving circuit is arranged to provide the driving signal comprising the first component at the first frequency and the second component at the second frequency; and wherein the first and the second frequency fall within the bandwidth of the resonant circuit.

11. A measurement system according to any of the claims 8 to 10, wherein the analyzer circuit comprises a frequency analyzer.

12. A measurement system according to any of the claims 8 to 11, wherein the analyzer circuit is configured to measure a ratio of the amplitudes of odd order intermodulation products resulting from the first and second components of the driving signal.

13. A measurement system according to any of the claims 8 to 12, further comprising a wire connection connecting the driving circuit and the measurement circuit.

14. A measurement system according to any of the claims 8 to 12, further comprising a transmitter arranged to wirelessly transmit the driving signal from the driving circuit to the measurement circuit.

15. A measurement system according to any of the claims 8 to 14, wherein the system is arranged to wirelessly transmit the response of the measurement circuit to the analyzer circuit.

16. A method of measuring a property in a body, the method comprising:

   applying a driving signal to a measurement circuit, wherein the driving signal comprises a first component at a first frequency and a second component at a second frequency, and wherein the measurement circuit comprises:

a resonant circuit comprising a first element and a second element, wherein at least one of the first element and the second element is configured such that a property thereof is dependent on the property in the body; and a non-linear element arranged to generate intermodulation products when the circuit is driven by an input signal comprising a first component at a first frequency and a second component at a second frequency; and analyzing a response of the measurement circuit in order to obtain a measurement of the property.

Figure 1

Figure 2

Figure 3

Figure 4

EP 3 406 187 A1

Figure 5

Figure 6

EP 3 406 187 A1

Figure 7

EP 3 406 187 A1

320

Apply driving signal to measurement circuit, wherein the driving signal comprises a first component at a first frequency and a second component at a second frequency

322

Analyze a response of the measurement circuit in order to obtain a measurement of the property

Figure 8

Europäisches
Patentamt

European
Patent Office

Office européen
des brevets

# EUROPEAN SEARCH REPORT

Application Number

EP 17 17 3013

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2004/014456 A2 (CARDIOMEMS INC [US]; ALLEN MARK [US]; FONSECA MICHAEL [US]; WHITE JASO) 19 February 2004 (2004-02-19) <br> * abstract; figures 1-22 * <br> * paragraphs [0014] - [0034], [0062] - [0075], [0096] - [0106] * | 1-16 | INV. <br> A61B5/00 <br> A61B5/0215 <br><br> ADD. <br> A61B5/053 |
| X | US 2003/037591 A1 (ASHTON TIM [GB] ET AL) 27 February 2003 (2003-02-27) <br> * abstract; figures 1-7 * <br> * paragraphs [0011], [0030] - [0036] * | 1-16 | |
| A | US 2010/277175 A1 (WEISS STEFFEN [DE]) 4 November 2010 (2010-11-04) <br> * abstract; figures 1-6 * <br> * paragraphs [0004] - [0006], [0011] - [0013], [0030] - [0040] * | 1-16 | |
| A | VILLE VIIKARI ET AL: "RFID MEMS Sensor Concept Based on Intermodulation Distortion", <br> IEEE SENSORS JOURNAL, IEEE SERVICE CENTER, NEW YORK, NY, US, <br> vol. 9, no. 12, <br> 1 December 2009 (2009-12-01), pages 1918-1923, XP011278923, <br> ISSN: 1530-437X, DOI: 10.1109/JSEN.2009.2031809 <br> * Abstract; Introduction; Theory of intermodulating sensor; Experiments and simulations; Utilization as a tag or sensor; Conclusion; <br> page 1918 - page 1922 * | 1-16 | |

TECHNICAL FIELDS
SEARCHED (IPC)

A61B

The present search report has been drawn up for all claims

| Place of search | Date of completion of the search | Examiner |
|---|---|---|
| The Hague | 9 November 2017 | Carta, Riccardo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 17 3013

This annex lists the patent family members relating to the patent documents cited in the above-mentioned European search report.
The members are as contained in the European Patent Office EDP file on
The European Patent Office is in no way liable for these particulars which are merely given for the purpose of information.

09-11-2017

| Patent document cited in search report | Publication date | Patent family member(s) | Publication date |
|---|---|---|---|
| WO 2004014456 A2 | 19-02-2004 | AU 2003265380 A1 | 25-02-2004 |
| | | AU 2009201120 A1 | 09-04-2009 |
| | | CA 2494989 A1 | 19-02-2004 |
| | | EP 1545303 A2 | 29-06-2005 |
| | | WO 2004014456 A2 | 19-02-2004 |
| US 2003037591 A1 | 27-02-2003 | JP 2003126126 A | 07-05-2003 |
| | | US 2003037591 A1 | 27-02-2003 |
| US 2010277175 A1 | 04-11-2010 | CN 101529266 A | 09-09-2009 |
| | | EP 2054733 A1 | 06-05-2009 |
| | | JP 2010517595 A | 27-05-2010 |
| | | US 2010277175 A1 | 04-11-2010 |
| | | WO 2008020375 A1 | 21-02-2008 |

EPO FORM P0459

For more details about this annex : see Official Journal of the European Patent Office, No. 12/82

**REFERENCES CITED IN THE DESCRIPTION**

**Patent documents cited in the description**

- WO 2012109039 A
- WO 2014164734 A
- WO 2015099845 A
- WO 2017013224 A
- WO 2016207041 A
- US 20130060139 A1